# EUROPÄISCHE PATENTSCHRIFT

(11) **EP 0 753 504 B1**
(45) Veröffentlichungstag und Bekanntmachung des Hinweises auf die Patenterteilung: **21.04.1999**
(21) Anmeldenummer: 96110782.8
(22) Anmeldetag: 04.07.1996
(51) Int. Cl.: C07C 209/12, C07C 211/63

(54) **Verfahren zur Herstellung von (2-Chlorethyl)-ammoniumchloriden**
Process for the preparation of (2-chloroethyl) ammonium chlorides
Procédé pour la préparation de chlorures de (2-chloroéthyl)-ammonium

(30) Priorität: 13.07.1995 DE 19525507
(43) Veröffentlichungstag der Anmeldung: 15.01.1997
(73) Patentinhaber: BASF AKTIENGESELLSCHAFT, 67056 Ludwigshafen (DE)
(72) Erfinder: Becker, Rainer, Dr., 67098 Bad Dürkheim (DE); Kersten, Siegfried, Dr., 67227 Frankenthal (DE); Ross, Karl-Heinz, Dr., 67269 Grünstadt (DE); Stadler, Klaus Peter, Dr., 67354 Römerberg (DE); Wache, Harro, Dr., 67136 Fussgönheim (DE); Wambach, Ludwig, Dr., 68723 Schwetzingen (DE); Weyer, Hans-Jürgen, Dr., 67240 Bobenheim-Roxheim (DE)

(56) Entgegenhaltungen:
- EP-A- 0 024 489
- DE-A- 1 493 469
- DE-A- 1 963 400
- DE-B- 1 275 066

## Beschreibung

Die vorliegende Erfindung betrifft ein Verfahren zur Herstellung von Chlorcholinchlorid aus Trimethylamin und 1,2-Dichlorethan durch eine spezielle Temperaturführung.

Zur Herstellung von Chlorcholinchlorid sind eine Reihe von Verfahren bekannt, doch sind für technische Belange nur solche interessant, die die Umsetzung von Trimethylamin und 1,2-Dichlorethan vorsehen. Hierbei ist zu berücksichtigen, daß Reaktionsbedingungen zu wählen sind, die eine Hydrolyse des 1,2-Dichlorethan weitgehend ausschließen. Es ist bekannt, daß deswegen vor allem Verfahren zur Anwendung kommen, bei denen die Umsetzung unter wasserfreien Bedingungen erfolgt. Die Synthese des Produktes wird überwiegend diskontinuierlich durchgeführt.

Aus J. Biol. Chemistry 235 (1960), Seite 475 bis 479, ist die Herstellung von Chlorcholinchlorid durch Umsetzung von Trimethylamin mit 1,2-Dichlorethan in Toluol bei einer Temperatur von 120°C bekannt. Nach einer Reaktionszeit von 7 Stunden sind erst etwa 80 % der Einsatzstoffe umgesetzt.

Verwendet man 1,2-Dichlorethan mit mindestens 4- bis 6-fachem Überschuß, bezogen auf wasserfreies Trimethylamin, dann läuft die Reaktion bei etwa 100°C unter Eigendruck innerhalb 1,5 bis 2 Stunden mit einer Ausbeute von 98 % ab (DE-A-12 75 066).

Nach DE-A-14 93 469 kann mit 1,2-Dichlorethan als Lösungsmittel auch bei höheren Temperaturen gearbeitet werden. So wird beispielsweise beschrieben, daß 1,2-Dichlorethan und Trimethylamin zusammen gegeben werden und die Mischung allmählich auf 130°C erhitzt und bei dieser Temperatur und einem Druck von maximal 6 bar für 3 Stunden umgesetzt wird (Ausbeute 93 %).

DE-A-19 63 400 beschreibt die Umsetzung von 1,2-Dichlorethan mit Trimethylamin in Gegenwart von 0,1 bis 2 Gew.-% Wasser bei Temperaturen von 60 bis 200°C. Die in Beispiel 2 beschriebene kontinuierliche Umsetzung von 1,2-Dichlorethan mit Trimethylamin bei 150°C und 6 bar führt zu einer Chlorcholinchlorid-Ausbeute von 99 %.

Chlorcholinchloridlösung wie auch das in fester Form gewonnene Chlorcholinchlorid enthalten noch 1,2-Dichlorethan und Trimethylamin bzw. Trimethylamin-Hydrochlorid. Beide Verbindungen können relativ einfach, beispielsweise durch Trocknen bzw. Andestillie-ren im Vakuum, weitgehend entfernt werden.

Problematischer ist hingegen das Nebenprodukt Trimethylvinylammoniumchlorid, das sich nach beendeter Reaktion nicht mehr vom Chlorcholinchlorid abtrennen läßt. So entsteht beispielsweise bei der Umsetzung von 1,2-Dichlorethan mit Trimethylamin im molaren Verhältnis 1:5 bei 100°C und einer Reaktionszeit von 2 Stunden ein Chlorcholinchlorid, das noch 1,4 Gew.-% Trimethylvinylammoniumchlorid enthält. Unter diesen Reaktionsbedingungen sinkt der Gehalt an Trimethylvinylammoniumchlorid erst bei beträchtlicher Verlängerung der Reaktionszeit. So liegt der Gehalt an Trimethylvinylammoniumchlorid erst nach einer Reaktionszeit von 4,5 Stunden unter 0,05 Gew.-%. Wird die Umsetzung von Beginn an bei höheren Temperaturen durchgeführt, entsteht durch die damit verbundenen höheren Drücke ein nicht unerhebliches Sicherheitsrisiko sowie ein deutlich erhöhter apparativer Aufwand.

Der vorliegenden Erfindung lag daher die Aufgabe zugrunde, den zuvor genannten Nachteilen abzuhelfen, insbesondere ein Verfahren zu finden, mit dem man in einfacher Weise unter Berücksichtigung der sicherheitstechnischen und wirtschaftlichen Aspekte bei gleichbleibend hoher Produktqualität die Reaktionszeit zu verkürzen.

Demgemäß wurde ein neues und verbessertes Verfahren zur Herstellung von Chlorcholinchlorid der Formel 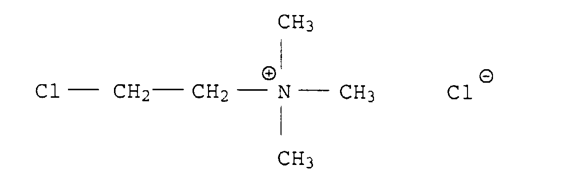 durch Umsetzung von 1,2-Dichlorethan mit Trimethylamin bei Temperaturen von 50 bis 200°C gefunden, welches dadurch gekennzeichnet ist, daß man 1,2-Dichlorethan mit Trimethylamin bei Temperaturen von 50 bis 100°C mischt und anschließend die Temperatur stufenweise oder fortlaufend auf 120 bis 200°C erhöht. Es ist sinnvoll, die Reaktionstemperatur erst dann zu erhöhen, wenn das Trimethylamin großenteils abreagiert hat, beispielsweise nachdem ein 85 %iger Amin-Umsatz erreicht ist.

Das erfindungsgemäße Verfahren kann wie folgt durchgeführt werden:
1,2-Dichlorethan mit 0 bis 2 Gew.-%, bevorzugt 0 bis 0,5 Gew.-%, besonders bevorzugt 0 bis 0,1 Gew.-% wasser und Trimethylamin können, gegebenenfalls vorgeheizt bei Temperaturen von 50 bis 100°C, bevorzugt 70 bis 100°C, besonders bevorzugt 90 bis 100°C, gegebenenfalls in einem inerten Lösungsmittel, gemischt werden. Anschließend, insbesondere nach Erreichen von 85%, bevorzugt 90%, besonders bevorzugt 95%, Trimethylamin-Umsatz kann stufenweise oder fortlaufend (kontinuierlich) die Temperatur auf 120 bis 200°C, bevorzugt 120 bis 160°C, besonders bevorzugt 120 bis 140°C, unter Eigendruck von ca. 1,5 bis 8 bar, bevorzugt 2 bis 6 bar, besonders bevorzugt 3 bis 5 bar, erhöht werden.

Das Molverhältnis von 1,2-Dichlorethan zum Trimethylamin beträgt in der Regel 0,8:1 bis 20:1, bevorzugt 1:1 bis 10:1, besonders bevorzugt 2:1 bis 6:1.

Die Umsetzung kann diskontinuierlich oder kontinuierlich durchgeführt werden.

Als inerte Lösungsmittel eignen sich aromatische Kohlenwasserstoffe wie Benzol, Toluol und die Xylole, bevorzugt Toluol, oder besonders bevorzugt ein Reaktionspartner, beispielsweise 1,2-Dichlorethan.

Überraschenderweise ist weder eine Verfärbung des Produkts noch eine Verringerung der Ausbeute zu beobachten.

Das Chlorcholinchlorid eignet sich als Regulator des Pflanzenwachstums (Antigilberelline) [Ullmanns Encyklopädie der technischen Chemie, Bd. 18, 3. Auflage, Hrsg. Dr. Dr. W. Foerst, München-Berlin-Wien 1969].

### Beispiele

### Beispiel 1

In einem druckfesten geschlossenen 1 1-Reaktor wurden 625 g 1,2-Dichlorethan bei 100°C vorgelegt und 67 g verflüssigtes Trimethylamin innerhalb 1 h zugegeben. Danach wurde 0,5 h 120°C und anschließend 0,5 h 140°C eingestellt. Der maximale Druck lag bei 3,8 bar. Nach Abkühlung auf 80°C wurden unter Rühren 111 g Wasser zugefügt. Durch Phasentrennung erhielt man eine wäßrige ca. 60 Gew.-% Chlorcholinchlorid-Lösung, die weniger als 0,05 Gew.-% Trimethylvinylammoniumchlorid enthielt.

### Beispiel 2

Die Umsetzung wurde analog Beispiel 1 durchgeführt, mit der Maßgabe, daß nach der Zugabe des Trimethylamins bei 100°C anschließend die Temperatur 1 h bei 140°C gehalten wurde. Der Gehalt an Trimethylvinylammoniumchlorid in der wäßrigen Chlorcholinchlorid-Lösung betrug weniger als 0,05 Gew.-%.

### Beispiel 3

Die Umsetzung wurde analog Beispiel 1 durchgeführt, mit der Maßgabe, daß nach der Zugabe des Trimethylamins bei 100°C anschließend die Temperatur 0,5 h bei 120°C und 1 h bei 140°C gehalten wurde. Der Gehalt an Trimethylvinylammoniumchlorid in der wäßrigen Chlorcholinchlorid-Lösung betrug weniger als 0,05 Gew.-%.

### Vergleichsbeispiel 4

Die Umsetzung wurde analog Beispiel 1 durchgeführt, mit der Maßgabe, daß nach der Zugabe des Trimethylamins bei 100°C anschließend die Temperatur 1 h bei 100°C gehalten wurde. Der Gehalt an Trimethylvinylammoniumchlorid in der wäßrigen Chlorcholinchlorid-Lösung betrug 1,4 Gew.-%.

### Vergleichsbeispiel 5

Die Umsetzung wurde analog Beispiel 1 durchgeführt, mit der Maßgabe, daß nach der Zugabe des Trimethylamins bei 100°C anschließend die Temperatur 2 h bei 100°C gehalten wurde. Der Gehalt an Trimethylvinylammoniumchlorid in der wäßrigen Chlorcholinchlorid-Lösung betrug 0,2 Gew.-%.

### Vergleichsbeispiel 6

Die Umsetzung wurde analog Beispiel 1 durchgeführt, mit der Maß-gabe, daß nach der Zugabe des Trimethylamins bei 100°C anschließend die Temperatur 3 h bei 100°C gehalten wurde. Der Gehalt an Trimethylvinylammoniumchlorid in der wäßrigen Chlorcholinchlorid-Lösung betrug weniger als 0,05 Gew.-%.

## Patentansprüche

1. Verfahren zur Herstellung von Chlorcholinchlorid durch Umsetzung von 1,2-Dichlorethan mit Trimethylamin bei Temperaturen von 50 bis 200°C, dadurch gekennzeichnet, daß man 1,2-Dichlorethan mit Trimethylamin bei Temperaturen von 50 bis 100°C mischt und anschließend die Temperatur stufenweise oder fortlaufend auf 120 bis 200°C erhöht.

2. Verfahren zur Herstellung von Chlorcholinchlorid nach Anspruch 1, dadurch gekennzeichnet, daß man bei Temperaturen von 70 bis 100°C mischt.

3. Verfahren zur Herstellung von Chlorcholinchlorid nach Anspruch 1, dadurch gekennzeichnet, daß man bei Temperaturen von 90 bis 100°C mischt.

4. Verfahren zur Herstellung von Chlorcholinchlorid nach Anspruch 1, dadurch gekennzeichnet, daß man die stufenweise oder fortlaufende Erhöhung der Temperaturen auf 120 bis 160°C durchführt.

5. Verfahren zur Herstellung von Chlorcholinchlorid nach Anspruch 1, dadurch gekennzeichnet, daß man die stufenweise oder fortlaufende Erhöhung der Temperaturen auf 120 bis 140°C durchführt.

6. Verfahren zur Herstellung von Chlorcholinchlorid nach den Ansprüchen 1 bis 4, dadurch gekennzeichnet, daß man die Reaktionstemperatur nach Erreichen von 85 % Trimethylamin-Umsatz erhöht.

7. Verfahren zur Herstellung von Chlorcholinchlorid nach den Ansprüchen 1 bis 4, dadurch gekennzeichnet, daß man die Reaktionstemperatur nach Erreichen von 90 % Trimethylamin-Umsatz erhöht.

8. Verfahren zur Herstellung von Chlorcholinchlorid nach den Ansprüchen 1 bis 4, dadurch gekennzeichnet, daß man die Reaktionstemperatur nach Erreichen von 95 % Trimethylamin-Umsatz erhöht.

9. Verfahren zur Herstellung von Chlorcholinchlorid nach den Ansprüchen 1 bis 5, dadurch gekennzeichnet, daß man die Umsetzung diskontinuierlich durchführt.

## Claims

1. A process for preparing chlorocholine chloride by reacting 1,2-dichloroethane with trimethylamine at from 50 to 200°C, which comprises mixing 1,2-dichloroethane with at from 50 to 100°C and subsequently raising the temperature to 120-200°C in stages or continuously.

2. A process for preparing chlorocholine chloride as claimed in claim 1, wherein the mixing step is carried out at from 70 to 100°C.

3. A process for preparing chlorocholine chloride as claimed in claim 1, wherein the mixing step is carried out at from 90 to 100°C.

4. A process for preparing chlorocholine chloride as claimed in claim 1, wherein the stagewise or continuous raising of the temperature is carried on to 120-160°C.

5. A process for preparing chlorocholine chloride as claimed in claim 1, wherein the stagewise or continuous raising of the temperature is carried on to 120-140°C.

6. A process for preparing chlorocholine chloride as claimed in any of claims 1 to 4, wherein the raising of the reaction temperature is carried out after the trimethylamine conversion is 85%.

7. A process for preparing chlorocholine chloride as claimed in any of claims 1 to 4, wherein the raising of the reaction temperature is carried out after the trimethylamine conversion is 90%.

8. A process for preparing chlorocholine chloride as claimed in any of claims 1 to 4, wherein the raising of the reaction temperature is carried out after the trimethylamine conversion is 95%.

9. A process for preparing chlorocholine chloride as claimed in any of claims 1 to 5, wherein the reaction is carried out batchwise.

## Revendications

1. Procédé de préparation de chlorures de chlorocholine par réaction de 1,2-dichloroéthane avec de la triméthylamine à des températures de 50-200°C, caractérisé en ce que l'on mélange du 1,2-dichloroéthane avec de la triméthylamine, à des températures de 50-100°C, puis on augmente la température par paliers ou de façon continue jusqu'à 120-200°C.

2. Procédé de préparation de chlorures de chlorocholine selon la revendication 1, caractérisé en ce que l'on mélange à des températures de 70-100°C.

3. Procédé de préparation de chlorures de chlorocholine selon la revendication 1, caractérisé en ce que l'on mélange à des températures de 90-100°C.

4. Procédé de préparation de chlorures de chlorocholine selon la revendication 1, caractérisé en ce que l'on augmente la température par paliers ou de façon continue jusqu'à 120-160°C.

5. Procédé de préparation de chlorures de chlorocholine selon la revendication 1, caractérisé en ce que l'on augmente la température par paliers ou de façon continue jusqu'à 120-140°C.

6. Procédé de préparation de chlorures de chlorocholine selon l'une quelconque des revendications 1 à 4, caractérisé en ce que l'on élève la température réactionnelle après avoir atteint un taux de conversion de la triméthylamine de 85%.

7. Procédé de préparation de chlorures de chlorocholine selon l'une quelconque des revendications 1 à 4, caractérisé en ce que l'on élève la température réactionnelle après avoir atteint un taux de conversion de la triméthylamine de 90%.

8. Procédé de préparation de chlorures de chlorocholine selon l'une quelconque des revendications 1 à 4, caractérisé en ce que l'on élève la température réactionnelle après avoir atteint un taux de conversion de la triméthylamine de 95%.

9. Procédé de préparation de chlorures de chlorocholine selon l'une quelconque des revendications 1 à 5, caractérisé en ce que l'on mène la réaction de façon discontinue.
